(19) Europäisches Patentamt
European Patent Office
Office européen des brevets

(11) Veröffentlichungsnummer: **0 105 267**
**B1**

(12) # EUROPÄISCHE PATENTSCHRIFT

(45) Veröffentlichungstag der Patentschrift: **10.12.86**

(51) Int. Cl.[4]: **C 07 D 309/12,** C 07 C 49/753, C 07 F 7/18

(21) Anmeldenummer: **82903041.0**

(22) Anmeldetag: **14.10.82**

(86) Internationale Anmeldenummer: **PCT/DE 82/00205**

(87) Internationale Veröffentlichungsnummer: **WO 83/01449 (28.04.83** Gazette **83/10)**

(54) **VERFAHREN ZUR HERSTELLUNG VON CARBACYCLIN-ZWISCHENPRODUKTEN.**

(30) Priorität: **23.10.81 DE 3142733**

(43) Veröffentlichungstag der Anmeldung: **18.04.84 Patentblatt 84/16**

(45) Bekanntmachung des Hinweises auf die Patenterteilung: **10.12.86 Patentblatt 86/50**

(84) Benannte Vertragsstaaten: **AT BE CH DE FR GB LI LU NL SE**

(56) Entgegenhaltungen:
**GB - A - 2 070 596**

**Angew. Chem. 92, 856 (1980)**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **VORBRÜGGEN, Helmut, Wilkestrasse 7, D-1000 Berlin 27 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft ein Verfahren zur Herstellung von geschützte Hydroxygruppen enthaltenden bicyclischen Ketonen aus entsprechenden Ketosäuren. Alle bekannten Verfahren zur Darstellung von Carbacyclin-Zwischenstufen, die von Zwischenprodukten der Corey'schen Prostaglandin-Synthese, insbesondere den γ-Lactonen, ausgehen, verlaufen nicht nur über viele Reaktionsstufen (vgl. DE-OS 2 912 409, DE-OS 3 021 895 oder GB 2 070 596, worin P. A. Aristoff ebenfalls eine mehrstufige Synthese von Verbindungen der Formel I beschreibt) und ergeben unerwünschte Nebenprodukte, sondern verlangen auch sehr genau einzuhaltende Reaktionsbedingungen (vgl. P. A. Aristoff, J. Org. Chem. 46, 1954 [1981]) für die ablaufende interne Wittig-Reaktion. Nach P. A. Aristoff halten z.B. Tetrahydropyranyl(THP)-geschützte Hydroxygruppen in γ-Ketoverbindungen schärferen Reaktionsbedingungen wie z.B. den von H. J. Bestmann et al. in Angew. Chemie 92, 856 (1980) beschriebenen, nicht stand. Es entstehen als Hauptprodukt nicht mehr die gewünschten Carbacyclin-Zwischenprodukte sondern durch β-Eliminierung des THP-Äthers im Cyclopentanring Verbindungen vom Typ des PGA. In Angew. Chem. 89, 361 (1977) werden Umsetzungen von Triphenyl- (phenyliminovinyliden)-phosphoran mit CH-aciden Verbindungen und Keto-Verbindungen, jedoch nicht mit γ-Oxocarbonsäuren beschrieben. Die Umsetzung mit γ-Oxocarbonsäuren wird von Bestmann erst später in Angew. Chem. 92, 856 (1980) erwähnt.

Es wurde gefunden, dass sich die aus Corey-Lactonen durch alkalische Öffnung und Jones-Oxydation der γ-Hydroxysäure leicht zugänglichen γ-Ketosäuren durch Umsetzung mit Triphenyl (phenyliminovinyliden)-phosphoran in guten Ausbeuten in die α,β-ungesättigten Ketone umwandeln lassen. Diese Ketone können durch Hydrierung oder Hydridübertragung mit Triäthylammoniumformiat/5% Palladium-Kohle (vgl. R. F. Heck et al., J. Org. Chem. 43, 3985 [1978]) sehr leicht in gesättigte Carbacyclin-Zwischenprodukte überführt werden.

Gegenstand der Erfindung ist somit ein Verfahren zur Herstellung von geschützten Hydroxygruppen enthaltenden bicyclischen Ketonen der allgemeinen Formel I

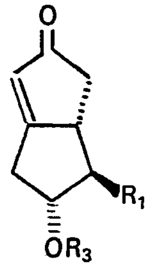

(I),

worin

$R_1$ die Reste $-CH_2OR_2$ mit $R_2$ als Benzyl oder Tetrahydropyranyl,

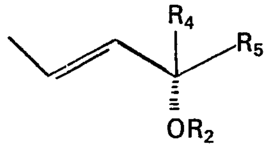

mit $R_2$ in der oben angegebenen Bedeutung,

$R_4$ als Wasserstoff oder Methyl und $R_5$ als geradkettiger oder verzweigtkettiger Alkylrest mit 1–10 C-Atomen, der durch Fluor, Chlor, 1,2-Methylen, 1,1-Trimethylen oder Methoxy substituiert sein kann, als Pentinyl oder als $CH_2$–X-Aryl-Rest, wobei X $CH_2$ oder 0 und Aryl Phenyl, oder

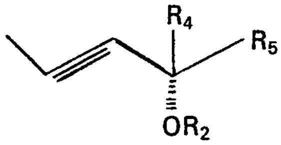

mit den für $R_2$, $R_4$ und $R_5$ angegebenen Bedeutungen, sein kann,

und $R_3$ die für $R_2$ angegebene Bedeutung hat und identisch mit $R_2$ oder verschieden von $R_2$ sein kann, dadurch gekennzeichnet, dass man eine geschützte Hydroxygruppen enthaltende Ketosäure der allgemeinen Formel II

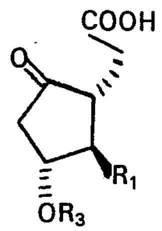

(II),

mit Triphenyl- (phenyliminovinyliden)-phosphoran und anschliessend mit Alkoholen umsetzt.

Die Reaktion der γ-Ketosäuren II mit Triphenyl-(phenyliminovinyliden)-phosphoran wird unter Feuchtigkeitsausschluss in aprotischen Lösungsmitteln wie Essigester, Toluol, Äthylenchlorid, Acetonitril, vorzugsweise in Essigester oder Acetonitril bei Temperaturen von 50–150 °C, in der Regel beim Siedepunkt des betreffenden Lösungsmittels in 1–5 h durchgeführt. Nachdem das Reaktionsgemisch eingeengt worden ist, schliesst sich ohne Aufreinigung die Umsetzung mit Alkoholen innerhalb von 5–20 Stunden in absoluten aprotischen Lösungsmitteln, vorzugsweise in Toluol an.

Als Alkohole für die genannte Reaktion kommen hauptsächlich n-Alkohole, mit 1–4 C-Atomen wie Methanol, Äthanol, n-Propanol, Isopropanol, n-Butanol in Betracht.

Als Alkylgruppen $R_5$ kommen gerad- und verzweigtkettige Alkylreste mit 1–10 C-Atomen in Frage, die gegebenenfalls Fluor, Chlor, 1,2-Methylen, 1,1-Trimethylen oder Methoxy substituiert sein können. Beispielsweise genannt seien Methyl-, Äthyl-, Propyl-, Butyl-, Isobutyl-, tert.-Butyl-, Pentyl-, Hexyl-, Heptyl-, Octyl-. Als weiterer Rest $R_5$ sei Pentinyl genannt.

Die nach dem erfindungsgemässen Verfahren hergestellten Carbacyclin-Zwischenprodukte stel-

len Ausgangsverbindungen für pharmakologisch wirksame Prostacycline dar.

Beispiel 1

7α- (Tetrahydropyran-2-yl-oxy) -6β- (benzyloxymethylen) - bicyclo-[3.3.0]- oct-1-en-3-on

3,5 g (ca. 10 mmol) rohes 2α-Carboxymethyl-3-β- benzyloxymethyl-4α- (tetrahydropyran-2-yl-oxy)- cyclopentanon wurden in 80 ml trockenem Essigester mit 3,8 (10 mmol) Triphenyl- (phenyliminovinyliden)- phosphoran unter Feuchtigkeitsauschluss 3 h gekocht, eingeengt und der Rückstand in 40 ml Toluol und 2,5 ml abs. Äthanol 12 h am Rückfluss gekocht. Das Rohprodukt wurde abgedampft und mehrfach unter Zusatz von Aceton heiss mit Hexan extrahiert, wobei 6,4 g roher Hexanextrakt erhalten wurde, der mit Toluol-Essigester 19:1 und 9:1 an 300 g Silicagel chromatographiert wurde, wobei neben 0,72 g (ca. 20%) Ausgangslacton 1,02 g (31,3% Gesamtausbeute) Endprodukt entstanden waren.

Das Ausgangsmaterial für die obige Titelverbindung wurde wie folgt hergestellt:

2α-Carboxymethyl-3β-benzyloxymethylen-3α-(tetrahydropyran-2-yl-oxy)- cyclopentanon

Eine Lösung von 3,67 g (9,53 mmol) 7α- (Tetrahydropyran-2-yl-oxy)6β- (benzyloxymethyl)-2-oxa-bicyclo [3.3.0]- octan-3-on und 35 ml 1 NaOH in 35 ml Äther wurden 2,5 h bei 25 °C gerührt, die wässrige Phase abgetrennt, mit Eis gekühlt und vorsichtig mit eiskalter Zitronensäure auf pH 4–5 angesäuert. Nach Extraktion mit 2×20 ml eiskaltem $CH_2Cl_2$ wurden schnell über wenig $Na_2SO_4$ filtriert und mit 40 ml Aceton verdünnt. Nach Kühlen auf –30 °C gab man während 10 min ca. 4 ml Jones-Reagenz dazu. Nach 2,5 h bei –30 °C wurden nochmals 1 ml Jones-Reagenz zugegeben und schliesslich mit 5 ml Isopropanol versetzt. Nach weiteren 5 min bei –30 °C wurden ca. 70 ml Eiswasser zugesetzt und mit 3×50 ml $CH_2Cl_2$ extrahiert. Nach Waschen mit ges. NaCl-Lösung, Trocknen ($Na_2SO_4$) und Einengen erhielt man so ca. 3,5 g Rohprodukt, das sofort weiterverarbeitet wurde.

Beispiel 2

7α-(Tetrahydropyran-2-yl-oxy) -6β- [3′α- (tetrahydropyran-2-yl-oxy)- 4′-methyl-trans-1′-octenyl] -bicyclo [3.3.0]oct-1-en-3-on

2,22 g (ca. 4,76 mmol) rohes 2α- (Carboxymethylen) -3β- [3′α-(tetrahydropyran-2-yl-oxy)-4′-methyl-trans-1′-octenyl] -4α- (tetrahydropyran-2-yl-oxy) -cyclopentanon wurden mit 1,97 g (5,23 mmol) Triphenyl- (phenyliminovinyliden)-phosphoran in 43 ml trockenem Essigester 3 h am Rückfluss gekocht, abgedampft und den Rückstand mit 25 ml Toluol und 1,5 ml Äthanol 12 h gekocht. Nach Abdampfen und Extraktion mit kochendem Hexan und wenig Aceton chromatographierte man den Rückstand (3,81 g) an 200 g Silicagel mit Toluol-Essigester 9:1 und 8:2, wobei 1,1 g (ca. 50% Gesamtausbeute) Endprodukt erhalten wurden.

Das Ausgangsmaterial für die obige Titelverbindung wurde wie folgt hergestellt:

2α-(Carboxymethylen)- 3β- [3′α- (tetrahydropyran- 2-yl-oxy)- 4′-methyl-trans- 1′-octenyl]-4α- (tetrahydropyran-2-yl-oxy)- cyclopentanon

Eine Lösung von 2,25 g (5 mmol) 7α- (Tetrahydropyran-2-yl-oxy) -6β- [3′α- (tetrahydropyran-2-yl-oxy) -4′-methyl-trans-1′-octenyl] -2-oxabicyclo [3.3.0] octan-3-on in 19 ml Äther wurde 18 h über Nacht mit 19 ml 1 N NaOH gerührt, die Ätherphase mit 2 ml 1 N NaOH gerührt und die alkalische Phase bei 0 °C vorsichtig mit ges. Zitronensäurelösung auf pH = 4–5 angesäuert. Nach Extraktion mit 2×10 ml eiskaltem Methylenchlorid und Trocknen ($Na_2SO_4$) wurde mit 21 ml Aceton verdünnt und bei –30 °C, 2,54 ml Jones-Reagenz tropfenweise zugegeben. Nach 2,5 h bei –30 °C setzte man 3 ml Isopropanol dazu, rührte noch 5 min bei –30 °C und versetzte mit ca. 40 ml Eiswasser. Nach Ausschütteln mit 3×50 ml $CH_2Cl_2$, Waschen mit ges. NaCl-Lösung und Trocknen ($Na_2SO_4$) erhielt man 2,22 g rohe Titelverbindung, die sofort weiterverarbeitet wurde.

Beispiel 3

7α- (Tetrahydropyran-2-yl-oxy) -6β- [3′α-(tetrahydropyran- 2-yl-oxy) -4′-phenoxy-trans- 1′-butenyl] -bicyclo[3.3.0]octan-1-en-3-on

2,63 g (ca. 5 mmol) 2α-(Carboxymethylen) 3β- [3′α- (tetrahydropyran-2-yl-oxy) -4′-phenoxy-trans-1′-butenyl] -4α- (tetrahydropyran-2-yl-oxy) -cyclopentanon wurden mit 2,07 g (5,5 mmol) Triphenyl- (phenyliminovinyliden)-phosphoran in 45 ml trockenem Essigester 72 h bei 24° belassen und dann 3 h unter Feuchtigkeitsausschluss am Rückfluss gekocht, abgedampft und der Rückstand in 23 ml abs. Toluol mit 1,43 ml Äthanol 12 h gekocht. Nach Abdampfen, Lösen in wenig Aceton und Verdampfen des Acetons mit überschüssigem Hexan erhielt man einen Hexan-Extrakt (3,52 g), der an 200 g Silicagel mit Toluol-Essigester chromatographiert wurde. Elution mit 9:1 und 8:2-Gemisch lieferte 0,96 g (41%) der Titelverbindung, die im System Toluol-Essigester 2:1 dünnschicht-chromatographisch einheitlich war.

Das Ausgangsmaterial für die obige Titelverbindung wurde wie folgt hergestellt:

2α-(Carboxymethylen) -3β- [3′α- (tetrahydropyran-2-yl-oxy) -4′-phenoxy-trans-1′-butenyl]-4α- (tetrahydropyran-2-yl-oxy) -cyclopentanon

Eine Lösung von 2,36 g (5 mmol) 7α- (Tetrahydropyran-2-yl-oxy) -6β- [3′α- (tetrahydropyran-2-yl-oxy) -4′-phenoxy-trans-1′-butenyl] -2-oxa-bicyclo[3.3.0]octan-3-on wurde in 19 ml Äther 17 h mit 18,55 ml 1 N NaOH gerührt, die Phasen getrennt und die Ätherphase mit wenig 1 N NaOH nachextrahiert. Die vereinigte alkalisch-wässrige Phase wurde bei 0° mit eiskalter Zitronensäurelösung auf pH = 4–5 angesäuert und die wässrige Lösung mit eiskaltem Methylenchlorid extrahiert. Nach Filtration der Methylenchloridextrakte über eine Schicht von $Na_2SO_4$ wurde mit ca. 20 ml Aceton verdünnt, auf –30°

gekühlt und 2,54 ml Jones-Reagenz zugetropft. Nach 2,5 h gab man bei −30° 3 ml Isopropanol hinzu und rührte noch 5 Min. Man gab ca. 50 ml Eiswasser zum Reaktionsgemisch und extrahierte die wässrige Phase mit 3 ×50 ml Methylenchlorid. Die organische Phase wurde mit wenig gesättigter NaCl-Lösung neutral gewaschen, getrocknet ($Na_2SO_4$) und abgedampft, wobei 2,63 g rohe Titelverbindung erhalten wurden.

**Patentanspruch**

Verfahren zur Herstellung von geschützte Hydroxygruppen enthaltenden bicyclischen Ketonen der allgemeinen Formel I

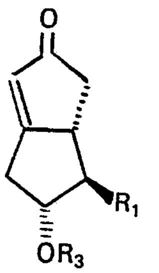

(I),

worin

$R_1$ die Reste $-CH_2OR_2$ mit $R_2$ als Benzyl oder Tetrahydropyranyl,

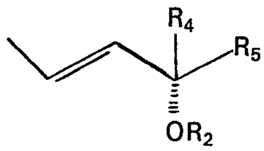 mit $R_2$ in der oben angegebenen Bedeutung,

$R_4$ als Wasserstoff oder Methyl und $R_5$ als geradkettiger oder verzweigtkettiger Alkylrest mit 1–10 C-Atomen, der durch Fluor, Chlor, 1,2-Methylen, 1,1-Trimethylen oder Methoxy substituiert sein kann, als Pentinyl oder als $CH_2$–X-Aryl-Rest, wobei X $CH_2$ oder 0 und Aryl Phenyl, oder

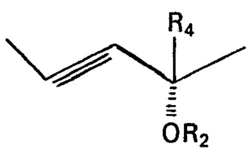 mit den für $R_2$, $R_4$ und $R_5$ angegebenen Bedeutungen, sein kann,

und $R_3$ die für $R_2$ angegebene Bedeutung hat und identisch mit $R_2$ oder verschieden von $R_2$ sein kann, dadurch gekennzeichnet, dass man eine geschützte Hydroxygruppen enthaltende Ketosäure der allgemeinen Formel II unter Feuchtigkeitsausschluss in einem aprotischen Lösungsmittel bei 50–150 °C

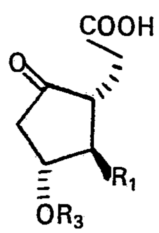

(II),

mit Triphenyl- (phenyliminovinyliden)- phosphoran und anschliessend mit einem Alkanol mit 1–4 C-Atomen in einem absoluten aprotischen Lösungsmittel umsetzt.

**Revendication**

Procédé de préparation de cétones bicycliques contenant des radicaux hydroxy protégés et répondant à la formule générale I:

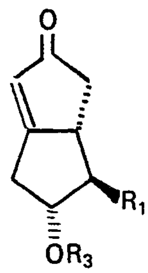

(I),

dans laquelle

$R_1$ représente:

– un radical $-CH_2OR_2$ dans lequel $R_2$ désigne un radical benzyle ou tétrahydropyrannyle,

– un radical 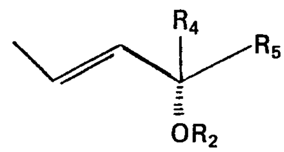 dans lequel:

$R_2$ a la signification précédemment donnée,

$R_4$ représente l'hydrogène ou un méthyle et

$R_5$ représente un radical alkyle en $C_1$–$C_{10}$, linéaire ou ramifié, qui peut porter un atome de fluor ou de chlore ou un radical méthylène-1,2, triméthylène-1,1 ou méthoxy, ou représente un radical pentynyle ou un radical $-CH_2$–X-aryl dans lequel X peut être $CH_2$ ou 0 et aryl un phényle, ou

– un radical 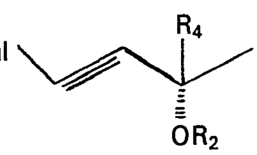 dans lequel:

$R_2$, $R_4$ et $R_5$ ont les significations précédemment données, et

$R_3$ a la signification qui a été donnée pour $R_2$ mais sans être obligatoirement identique à $R_2$, procédé caractérisé en ce qu'on fait, réagir un oxo-acide contenant des radicaux hydroxy protégés et répondant à la formule générale II:

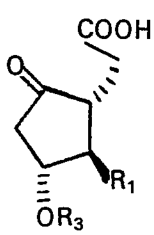

(II),

à l'abri de l'humidité, dans un solvant aprotique, à une température de 50 à 150 °C, avec le triphényl-(phényliminovinylidène) -phosphorane, puis avec un alcanol en $C_1$–$C_4$ dans un solvant aprotique anhydre.

**Claim**

Process for the preparation of bicyclic ketones containing protected hydroxy groups, of the general formula I

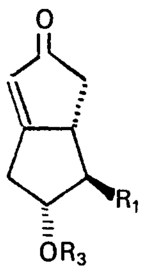

(I),

wherein

$R^1$ can be the group $-CH_2OR_2$, where $R_2$ is benzyl or tetrahydropyranyl,

the group 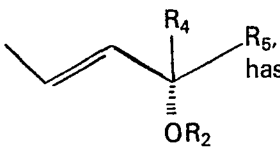 , where $R_2$ has the meaning given above,

$R_4$ is hydrogen or methyl and $R_5$ is a straight or branched chain alkyl group of 1–10 C-atoms which can be substituted by fluorine, chlorine, 1,2-methylene, 1,2-trimethylene or methoxy, or is pentynyl or a $CH_2$–X-aryl group, where X is $CH_2$ or 0 and aryl is phenyl, or

the group 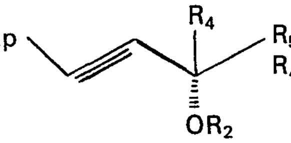 , where $R_2$, $R_4$ and $R_5$ have the meanings given above, and $R_3$ has the meaning given above for $R_2$ and can be the same as or different from $R_2$, characterised in that a keto acid containing a protected hydroxy group of general formula II

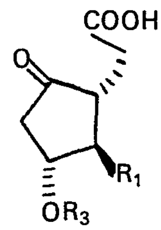

(II)

is reacted, under moisture free conditions, with triphenyl (phenyliminovinylidene) phosphorane in an aprotic solvent at 50–150 °C, followed by an alkanol of 1–4 C-atoms in an absolute aprotic sovent.